# EUROPEAN PATENT SPECIFICATION

(11) **EP 3 723 760 B1**
(45) Date of publication and mention of the grant of the patent: **11.06.2025**
(21) Application number: 18889552.8
(22) Date of filing: 13.12.2018
(51) Int. Cl.: A61K 9/00, A61K 9/10, A61K 9/14, A61K 31/519

(54) **ONE STEP MILLING PROCESS FOR PREPARING MICRONIZED PALIPERIDONE ESTERS**
EINSTUFIGES MAHLVERFAHREN ZUR HERSTELLUNG VON MIKRONISIERTEN PALIPERIDONSÄUREESTERN
PROCÉDÉ DE BROYAGE EN UNE ÉTAPE POUR PRÉPARER DES ESTERS DE PALIPÉRIDONE MICRONISÉS

(30) Priority: 14.12.2017 US 201762598827 P
(43) Date of publication of application: 21.10.2020
(73) Proprietor: SpecGx LLC, Webster Groves, Missouri 63119 (US)
(72) Inventor: SANDERS, Williams, Webster Groves, Missouri 63119 (US); LIU, Demin, Webster Groves, Missouri 63119 (US); YUE, Baohua, Webster Groves, Missouri 63119 (US)
(74) Representative: Jones, Nicholas Andrew
(86) International application number: PCT/US2018/065443
(87) International publication number: WO 2019/118722

(56) References cited:
- WO-A1-2016/157061
- CN-A- 106 137 985
- US-A1- 2004 089 753
- US-B2- 9 320 707
- LENG DONGLEI ET AL: "Development and comparison of intramuscularly long-acting paliperidone palmitate nanosuspensions with different particle size", INTERNATIONAL JOURNAL OF PHARMACEUTICS, ELSEVIER, NL, vol. 472, no. 1, 2 June 2014 (2014-06-02), pages 380 - 385, XP029037403, ISSN: 0378-5173, DOI: 10.1016/J.IJPHARM.2014.05.052

## Description

### FIELD OF THE INVENTION

The present disclosure generally relates to a process for preparing micronized paliperidone esters.

### BACKGROUND OF THE INVENTION

Paliperidone palmitate is an atypical antipsychotic agent belonging to the chemical class of benzisoxazole derivatives. The chemical name for paliperidone palmitate is 3-(2-(4-(6-fluoro-3a,7a-dihydrobenzo[d]isoxazol-3-yl)piperidin-1-yl)ethyl)-2-methyl-4-oxo-6,7,8,9-tetrahydro-4H-pyrido[1,2-a]pyrimidin-9-yl palmitate and is represented by the following formula:

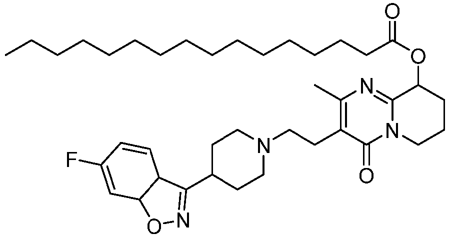

Currently, paliperidone palmitate is approved by the US Food and Drug Administration and the European Medicines Agency for the treatment of schizophrenia or a schizoaffective disorder. Additionally, paliperidone palmitate can be used to treat mania and at lower doses as maintenance for bipolar disorders.

Paliperidone palmitate can be formulated for parenteral administration. It was previously discovered that the particle size distribution of paliperidone palmitate determines the extended release kinetics of the formulations. Currently, paliperidone palmitate is micronized using air-jet mills or a multi-step wet milling process. Generally, sterile air-jet milling is carried out in isolators or by using a restricted access barrier system.

Paliperidone palmitate has been previously formulated for an extended-release suspension for intramuscular injection. The extended-release formulation is the result of the suspension having a range of paliperidone palmitate particle sizes. The range of paliperidone palmitate particle sizes is due to a multi-step wet milling process. The multi-step wet milling process requires the use of at least two different sized milling media, wherein a filtration or separation step is required to remove the first milling media before addition of the second milling media.

WO 2016/157061 A discloses a process for micronizing paliperidone palmitate by wet milling utilizing two types of milling beads sequentially. In comparative examples a single milling step is used.

Thus, there is a need to develop a manufacturing process that is more controlled than prior methods.

### SUMMARY OF THE INVENTION

The present disclosure provides a one step process for preparing a micronized paliperidone ester, the process comprising wet milling for 5 minutes ±5% to 60 minutes ±5% a suspension comprising a solid paliperidone ester having an average particle size from 10 µm ±5% to 200 µm ±5%, at least one suspending agent, and at least one wetting agent in the presence of a plurality of polymeric beads to form the micronized paliperidone ester, wherein the plurality of polymeric beads has an average diameter from 0.5 mm ±5% to 1.5 mm ±5%, the micronized paliperidone ester has a particle size distribution from 1 µm ±5% to 30 µm ±5%, and wherein the at least one suspending agent is polyethylene glycol, polypropylene glycol, or combinations thereof.

Other aspects and iterations of the disclosure are described in more detail below.

### BRIEF DESCRIPTION OF THE FIGURES

**FIG. 1** depicts particle size distribution of polystyrene milled paliperidone palmitate particles at different milling time points.
**FIG. 2** depicts dissolution profile of polystyrene milled paliperidone palmitate particles at different milling time points using a USP type 2 apparatus.

### DETAILED DESCRIPTION OF THE INVENTION

The present disclosure provides injectable pharmaceutical compositions that provide controlled release of an active pharmaceutical ingredient. Also provided are methods of making the controlled release pharmaceutical composition. The methods comprise a one-step wet milling process to provide a particle size distribution of the active pharmaceutical ingredient to obtain a controlled release formulation. The pharmaceutical compositions disclosed herein comprise at least one wetting agent, at least one suspending agent, and at least one active pharmaceutical ingredient. The pharmaceutical composition may further comprise at least one buffer and at least one pH modifier. Applicants of the present invention discovered that the milling of the active pharmaceutical ingredient with the other components, specifically, a wetting agent and a suspending agent, provides better control of the milling process which in turn provides better control of particle size distribution of the active pharmaceutical ingredient and prevents the generation of fine particles. The pharmaceutical compositions disclosed herein are milled in a one step process using polymeric beads. The resulting pharmaceutical composition provides better control of the controlled release profile.

### (I) Process for Preparing Micronized Paliperidone Ester

One aspect of the disclosure encompasses a process for preparing micronized paliperidone ester. The process is defined in the claims.

### (a) Forming the Suspension

The first step of the process comprises forming a suspension comprising a solid paliperidone ester, at least one suspending agent, and at least one wetting agent. The suspending agent is polyethylene glycol, polypropylene glycol, or combinations thereof. The process for forming the suspension generally takes place under aseptic conditions. Such methods are generally known in the art.

### (i) Active Pharmaceutical Ingredient (API)

The suspension comprises a paliperidone ester. In further embodiments, the paliperidone ester is paliperidone palmitate.

The API may be in any of its crystalline, semi-crystalline, amorphous, or polymorphous forms. In an exemplary embodiment, the API may be in a crystalline form.

The crystalline, semi-crystalline, amorphous, or polymorphous forms may have a particle size of about 10 to about 200 µm. In some embodiments, the crystalline, semi-crystalline, amorphous, or polymorphous forms may have a particle size of about 10 µm, about 20 µm, about 30 µm, about 40 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm.

The amount of API in the suspension can and will vary depending upon the active agent. In certain embodiments, the API in the suspension is paliperidone palmitate. In certain embodiments, the amount of paliperidone palmitate before milling may range from about 3% to about 60% w/v, from about 4% to about 50% w/v, from about 5% to about 40% w/v, from about 6% to about 35% w/v, from about 7% to about 30% w/v, from about 8% to about 25% w/v, or from about 10% to about 15% w/v of the suspension. In certain embodiments, the amount of paliperidone palmitate in the suspension may be about 5%, about 8%, about 10%, about 12%, about 14%, about 16%, about 18%, about 20%, about 25%, about 30%, or about 35% w/v of the suspension.

### (ii) Suspending Agent

The suspension comprises at least one suspending agent. In general, the at least one suspending agent provides long-term stabilization, facilitate drug adsorption, alter viscosity, or enhance solubility.

The suspending agent is polyethylene glycol, polypropylene glycol, or combinations thereof (*e.g*., PEG 300, PEG 400, PEG 600, PEG 1000, PEG 1100, PEG 1900, PEG 2000, PEG 2800, PEG 2900, PEG 3350, PEG 4000, PEG 6000, PEG 8000, PEG 8400, PEG 10,000, PEG 12,000, PEG 14600, PEG 17,000, etc.). In an exemplary embodiment, the suspending agent may be polyethylene glycol 4000 (PEG 4000).

The amount of the at least one suspending agent present in the suspension can and will vary depending upon the identity of the suspending agent as well as the identity and/or amount of the other components in the suspension. In general, the amount of the at least one suspending agent in the suspension may range from about 2% to about 30% w/v, from about 3% to about 27% w/v, from about 4% to about 23% w/v, from about 5% to about 20% w/v, from about 7% to about 17% w/v, or from about 10% to about 14% w/v of the suspension. In some embodiments, the amount of the at least one suspending agent in the suspension may be about 5%, about 6%, about 7%, about 8%, about 9%, about 10%, about 11%, about 12%, about 13%, about 14%, about 15%, about 16%, about 17%, about 18%, about 19%, or about 20% w/v of the suspension.

### (iii) Wetting Agent

The suspension also comprises at least one wetting agent. The at least one wetting agent can comprise amphiphilic compounds that contain polar, hydrophilic moieties as well as non-polar hydrophobic moieties. The wetting agent can be an anionic, cationic, a zwitterionic, or a non-ionic wetting agent.

Suitable wetting agents include, without limit, polyethylene glycol fatty acid monoesters (*e.g*., polyethylene glycol laurate or stearate, etc.); polyethylene glycol fatty acid diesters (*e.g*., polyethylene glycol dilaurate, disterate, dipalmitate, or dioleate, etc.); polyethylene glycol glycerol fatty acid esters (*e.g*., polyethylene glycol glycerol laurate, glycerol stearate, glycerol oleate, etc.); polyglycerized fatty acids (*e.g*., polyglyceryl laurate, oleate, or stearate; polyglyceryl mono and dioleate; etc.); sterol derivatives (*e.g*., polyethylene glycol cholesterol ether, polyethylene glycol cholestanol, polyethylene glycol phyto sterol, etc.); and polyethylene glycol sorbitan fatty acid esters (*e.g*., polysorbate 20, polysorbate 80, PEG-10 sorbitan laurate, PEG-20 sorbitan monolaurate, sorbitan tristearate, etc.). In an exemplary embodiment, the wetting agent may be polysorbate 20.

The amount of the at least one wetting agent present in the suspension can and will vary depending upon the identity of the wetting agent as well as the identity and/or amount of the other components utilized in the suspension. In general, the amount of the at least one wetting agent in the suspension may range from about 0.1% to about 10% w/v, from about 0.3% to about 8% w/v, from about 0.7% to about 5% w/v, or from about 1% to about 3% w/v of the suspension. In some embodiments, the amount of the at least one wetting agent in the suspension may be about 0.5%, about 1.0%, about 1.5%, about 2%, about 2.5%, about 3.0%, about 3.5%, about 4.0%, about 4.5%, or about 5% w/v of the suspension.

### (b) Additional Components

### (i) Buffer

The suspension may comprise at least one buffer. The identity of the buffer can and will vary depending on the pH of the suspension. Non-limiting examples of the buffer include the sodium or potassium salt of phthalate, phosphate, borate, and acetate. In an exemplary embodiment, the buffer may comprise sodium dihydrogen phosphate and/or disodium hydrogen phosphate.

In general, the amount of the at least one buffer in the suspension may range from about 0.1% to about 10% w/v, from about 0.2% to about 8% w/v, from about 0.3% to about 6% w/v, from about 0.4% to about 5% w/v, or from about 0.5% to about 3% w/v of the suspension. In certain embodiments, the amount of buffer in the suspension may be about 0.5%, about 1.0%, about 1.5%, about 2.0%, about 2.5%, or about 3.0% w/v of the suspension.

### (ii) pH Modifier

The suspension may comprise at least one pH modifier to adjust the pH of the suspension. Non-limiting examples of a pH modifier include organic or inorganic acids and bases, for example, acetic acid, citric acid, benzoic acid, formic acid, fumaric acid, hydrochloric acid, lactic acid, malic acid, phosphoric acid, sorbic acid, sulfuric acid, tartaric acid, potassium carbonate, sodium carbonate, sodium bicarbonate, and sodium hydroxide. In certain embodiment, the pH modifier may comprise sodium hydroxide and citric acid. In a specific embodiment the pH modifier may be citric acid.

In general, the amount of the at least one pH modifier in the suspension may range from about 0.1% to about 10% w/v, from about 0.2% to about 8% w/v, from about 0.3% to about 6% w/v, from about 0.4% to about 5% w/v, or from about 0.5% to about 3% w/v of the suspension. In certain embodiments, the amount of pH modifier in the suspension may be about 0.5%, about 1.0%, about 1.5%, about 2.0%, about 2.5%, or about 3.0% w/v of the suspension.

The pH of the suspension may range from about 6 to about 9, from about 7 to about 8, or from about 7 to about 7.5. In certain embodiment, the pH of the suspension may be about 7.0, about 7.1, about 7.2, about 7.3, about 7.4, or about 7.5.

In an embodiment, forming the suspension is carried in a batch or continuous process. In an exemplary embodiment, forming the suspension is carried out in a continuous process.

### (c) Wet Milling

The process further comprises milling the suspension to form micronized paliperidone ester. The process comprises milling the suspension to reduce particle size and generate a size distribution of micronized paliperidone ester particles. The milling process can be accomplished by techniques generally known in the art. Further, the milling process generally takes place under aseptic conditions.

The milling process generally utilizes milling media. The milling media comprise polymeric beads. Non-limiting examples of polymeric beads include polystyrene, polyamide, polycarbonate, acrylic, crosslinked polystyrene, crosslinked polyamide, or crosslinked polycarbonate. In an exemplary embodiment, the milling media is polystyrene beads crosslinked with divinylbenzene.

The size of the polymeric beads may vary depending upon the desired particle distribution. In some embodiments, the size of the polymeric beads may range from about 0.2 mm to about 2.0 mm. In various embodiments, the size of the polymeric beads may range from about 0.5 mm to about 1.5 mm, from about 0.5 mm to about 1.0 mm, or from about 0.6 to about 0.8 mm.

The milling process reduces the size of the paliperidone ester to the desired particle size distribution. The milling process is preferably accomplished so that the particle size distribution provides satisfactory dosage content uniformity, dissolution profiles, and a controlled release profile. The particle size distribution ranges from about 1 µm to about 30 µm or from about 2 µm to about 20 µm. In some embodiments, the d10 is from about 2 µm to about 3 µm, d50 is from about 6 µm to about 8 µm, and d90 is from about 15 µm to about 20 µm.

The time of the milling step can and will vary depending upon the components of the suspension. Additionally, the milling time may vary depending on the total amount of suspension prepared in **Section (I)(a).** The milling step proceeds for about 5 minutes to about 60 minutes, or from about 10 minutes to about 30 minutes.

The temperature of the milling step may also vary depending upon the identity of the API. In general, the milling step may occur at a temperature ranging from about 5°C to about 40°C. In specific embodiments, the milling step may occur at room temperature or about 20-25 °C.

The tip speed of the milling step can and will vary depending upon the mill utilized, media milling size, and final particle size distribution. In general, the milling speed may occur at from about 2 m/s to about 8 m/s. In further embodiments, the milling speed may occur at from about 3 m/s to about 6 m/s.

Following the milling process, the milling media is removed by standard techniques known in the art, and the micronized paliperidone ester suspension is diluted to the desired API concentration.

In an embodiment, the milling process is carried in a batch or continuous process. In an exemplary embodiment, the milling process is carried out in a continuous process.

### (d) Scale

The process of preparing a micronized palmitate ester can be conducted at a bench top (*e.g.*, 1 g to 100 g) to commercial scale (*e.g.*, 0.5 to 1000 kg).

### (II) Micronized Palmitate Ester

One aspect of the present disclosure provides a pharmaceutical composition that can be formulated to provide a controlled release of a micronized palmitate ester. Detailed below are the components and dosage forms of the pharmaceutical composition.

### (a) Components of the Pharmaceutical Composition

The pharmaceutical composition disclosed herein comprises at least one wetting agent, at least one suspending agent, at least one buffer, at least one pH modifier, and at least one active pharmaceutical ingredient. The order of component addition and the one-step milling process provide the desired API particle distribution which creates a controlled release formulation.

In an exemplary embodiment, the pharmaceutical composition comprises paliperidone palmitate, polysorbate 20, polyethylene glycol 4000, citric acid monohydrate, sodium dihydrogen phosphate, sodium hydroxide, and water for injection. In further exemplary embodiment, the pharmaceutical composition comprises paliperidone palmitate in an amount from about 25% to about 40% by weight of the composition, polyethylene glycol 4000 in an amount from about 5% to about 10% by weight of the composition, polysorbate 20 in an amount from about 0.5% to about 2% by weight of the composition, citric acid in an amount from about 0.1% to about 1.0% by weight of the composition, sodium dihydrogen phosphate in an amount from about 0.1% to about 1.0% by weight of the composition, sodium hydroxide in an amount from about 0.1% to about 1.0% by weight of the composition, and has a pH of about 7 to about 7.5.

In an additional exemplary embodiment, the pharmaceutical composition comprises paliperidone palmitate in an amount of about 312 mg/mL, polysorbate 20 in an amount of about 10 mg/mL, polyethylene glycol 4000 in an amount of about 75 mg/mL, citric acid in an amount of about 7.5 mg/mL, sodium dihydrogen phosphate in an amount of about 6 mg/mL, sodium hydroxide in an amount 5.4 mg/mL, and has a pH of about 7 to about 7.5.

### (b) Dosage Forms

The physical form of the pharmaceutical composition disclosed herein can and will vary. In general, the pharmaceutical composition can be prepared for parenteral administration.

Preparations for parenteral administration may be in a suspension. For parenteral administration (including subcutaneous, intradermal, intramuscular, and intraperitoneal), the preparation may be an aqueous or an oil-based suspension. Aqueous suspensions may include a sterile diluent such as water, saline solution, a pharmaceutically acceptable polyol such as glycerol, propylene glycol, or other synthetic solvents; an antibacterial and/or antifungal agent such as benzyl alcohol, methyl paraben, chlorobutanol, phenol, thimerosal, and the like; an antioxidant such as ascorbic acid or sodium bisulfite citrate; a chelating agent such as etheylenediaminetetraacetic acid; a buffer such as acetate, citrate, or phosphate; and/or an agent for the adjustment of tonicity such as sodium chloride, dextrose, or a polyalcohol such as mannitol or sorbitol. Oil-based suspensions may further comprise sesame, peanut, olive oil, or mineral oil. In an exemplary embodiment, the pharmaceutical composition is formulated as an injectable suspension.

### DEFINITIONS

Compounds useful in the compositions and methods include those described herein including diastereomers and enantiomers, as well as racemic mixtures and pure isomers of the compounds described herein, where applicable.

When introducing elements of the present invention or the preferred embodiments(s) thereof, the articles "a," "an," "the," and "said" are intended to mean that there are one or more of the elements. The terms "comprising," "including" and "having" are intended to be inclusive and mean that there may be additional elements other than the listed elements.

The term "about," particularly in reference to a given quantity, is meant to encompass deviations of plus or minus five percent.

The term "aseptic," as used herein, refers to aseptic processing or manufacturing that complies with Good Manufacturing Practice (GMP) industry guidelines such as those associated with Guidance for Industry-Sterile Drug Products Produced by Aseptic Processing-Current Good Manufacturing Practice, U.S. Department of Health and Human Services Food and Drug Administration.

### EXAMPLES

The following examples are included to demonstrate preferred embodiments of the invention. It should be appreciated by those of skill in the art that the techniques disclosed in the examples represent techniques discovered by the inventors to function well in the practice of the invention. ,

### Example 1: Preparation of Suspension

Polysorbate 20 (8.06 g), PEG 4000 (60.64 g), citric acid monohydrate (6.08 g), sodium dihydrogen phosphate (4.85 g), and sodium hydroxide (4.39 g) were weighed and transferred to a volumetric flask. Water (500 mL) for injection was added to the excipient mixture in a volumetric flask, mixed until completely dissolved, and then filtered.

Paliperidone palmitate (63 g) was weighed and transferred to a glass bottle. The filtered excipient solution was added to the paliperidone palmitate and mixed to create a suspension at the targeted milling concentration (6-35 w/v%). The mixture was stirred for an hour to thoroughly wet paliperidone palmitate to produce a homogenous suspension.

### Example 2: One Step Manufacturing Process

The suspension from Example 1 (6-35 w/v%) was milled in a NETZSCH MINICER bead mill using 700 micron polystyrene grinding media (40-85% media load). The mixture was milled at a speed of 3.1-5.5 m/s at 15-25°C for 20 minutes.

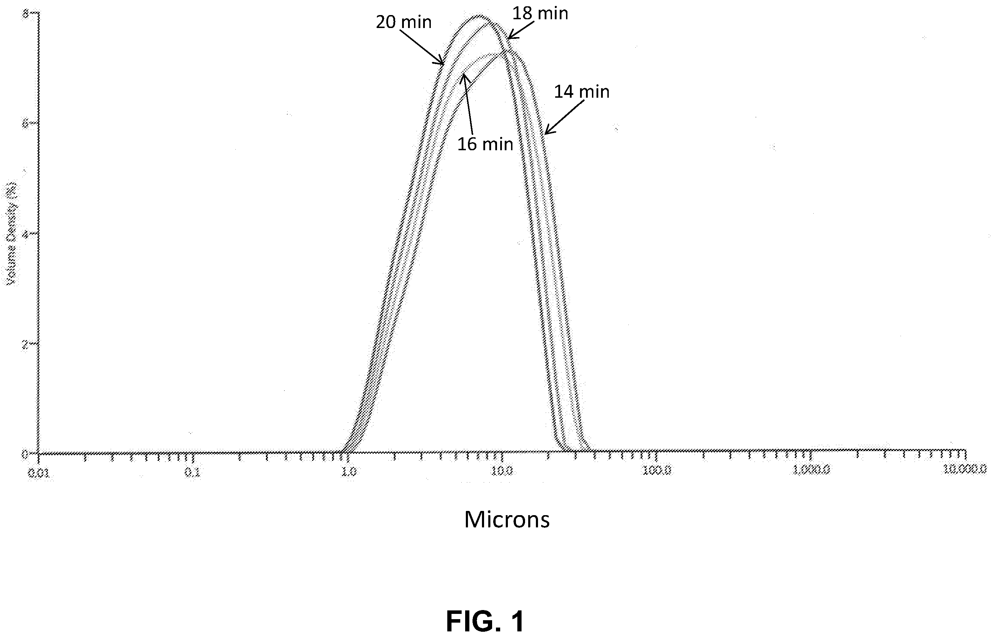

Following milling, particle size was measured, in deionized water, using a MALVERN MASTERSIZER 3000 particle size analyzer. The mean milled paliperidone palmitate particle size distribution of the suspension was D90 less than 20 micron, D50 less than 10 micron, and a D10 less than 3 micron **(****FIG. 1****).**

### Example 3: Dissolution Profile on One-Step Milled Particle

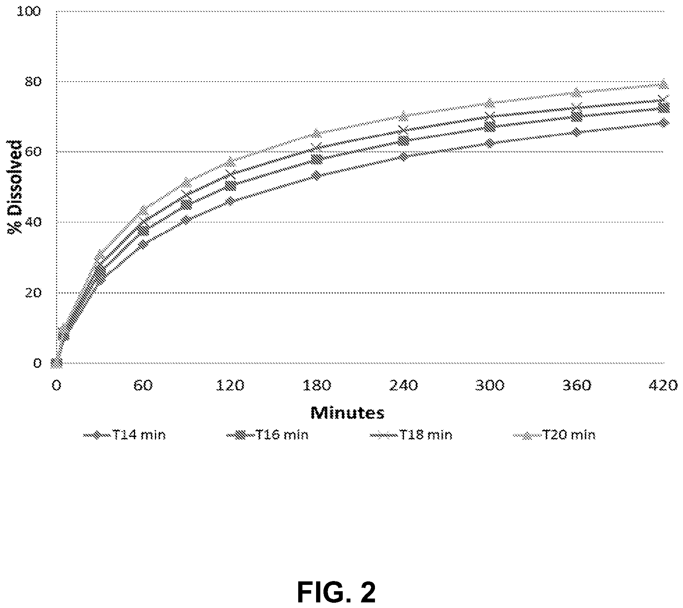

Paliperidone palmitate release from the milled particles prepared in Example 2 was examined using a USP type 2 apparatus. The release profile is presented in FIG. 2.

## Claims

1. A one step process for preparing a micronized paliperidone ester, the process comprising wet milling for 5 minutes ±5% to 60 minutes ±5% a suspension comprising a solid paliperidone ester having an average particle size from 10 µm ±5% to 200 µm ±5%, at least one suspending agent, and at least one wetting agent in the presence of a plurality of polymeric beads to form the micronized paliperidone ester, wherein the plurality of polymeric beads has an average diameter from 0.5 mm ±5% to 1.5 mm ±5%, the micronized paliperidone ester has a particle size distribution from 1 µm ±5% to 30 µm ±5%, and wherein the at least one suspending agent is polyethylene glycol, polypropylene glycol, or combinations thereof.

2. The process of claim 1, wherein the solid paliperidone ester is paliperidone palmitate.

3. The process of claim 1 or claim 2, wherein the at least one wetting agent is polyethylene glycol laurate, glycerol laurate, glycerol stearate, glycerol oleate, polysorbate 20, polysorbate 80, or combinations thereof.

4. The process of claim 1, wherein the solid paliperidone ester is paliperidone palmitate, the at least one suspending agent is polyethylene glycol 4000, and the at least one wetting agent is polysorbate 20.

5. The process of any one of claims 1 to 4, wherein the suspension comprises 3% (w/v) ±5% to 60% (w/v) ±5% of the solid paliperidone ester, 2% (w/v) ±5% to 30% (w/v) ±5% of the at least one suspending agent, and 0.1% (w/v) ±5% to 10% (w/v) ±5% of the at least one wetting agent.

6. The process of any one of claims 1 to 5, wherein the plurality of polymeric beads consists of polystyrene beads crosslinked with divinylbenzene, and the plurality of polymeric beads is present in amount from 40% (w/v) ±5% to 85% (w/v) ±5%.

7. The process of any one of claims 1 to 6, wherein the plurality of polymeric beads has an average diameter from 0.6 mm ±5% to 0.8 mm ±5%.

8. The process of any one of claims 1 to 7, wherein the suspension further comprises at least one buffer chosen from sodium dihydrogen phosphate, disodium hydrogen phosphate, sodium acetate, sodium phthalate, or combinations thereof, and the at least one buffer is present in an amount from 0.1% (w/v) ±5% to 10% (w/v) ±5%.

9. The process of any one of claims 1 to 8, wherein the suspension further comprises at least one pH modifier chosen from hydrochloric acid, citric acid, sodium hydroxide, or combinations thereof, and the at least one pH modifier is present in an amount from 0.1% (w/v) ±5% to 10% (w/v) ±5%.

10. The process of any one of claims 1 to 9, wherein the micronized paliperidone ester has a particle size distribution in which d10 is from 2 µm ±5% to 3 µm ±5%, d50 is from 6 µm ±5% to 8 µm ±5%, and d90 is from 15 µm ±5% to 20 µm ±5%.

11. The process of any one of claims 1 to 10, wherein the process is carried out as a continuous process.

12. The process of any one of claims 1 to 11, wherein the process is conducted aseptically.

13. The process of any one of claims 1 to 12, further comprising formulating the micronized paliperidone ester into an injectable dosage form.

## Patentansprüche

1. Einstufiges Verfahren zum Herstellen eines mikronisierten Paliperidonesters, wobei das Verfahren das Nassmahlen einer Suspension, die einen festen Paliperidonester mit einer durchschnittlichen Teilchengröße von 10 µm ±5% bis 200 µm ±5%, mindestens ein Suspendiermittel und mindestens ein Benetzungsmittel umfasst, für 5 Minuten ±5% bis 60 Minuten ±5% in Gegenwart einer Vielzahl von Polymerkügelchen umfasst, um den mikronisierten Paliperidonester zu bilden, wobei die Vielzahl der Polymerkügelchen einen durchschnittlichen Durchmesser von 0,5 mm ±5 % bis 1,5 mm ± 5 % aufweist, der mikronisierte Paliperidonester eine Teilchengrößenverteilung von 1 µm ±5 % bis 30 µm ±5 % aufweist und wobei das mindestens eine Suspendiermittel Polyethylenglykol, Polypropylenglykol oder Kombinationen davon ist.

2. Verfahren nach Anspruch 1, wobei der feste Paliperidonester Paliperidonpalmitat ist.

3. Verfahren nach Anspruch 1 oder 2, wobei das mindestens eine Benetzungsmittel Polyethylenglykollaurat, Glycerinlaurat, Glycerinstearat, Glycerinoleat, Polysorbat 20, Polysorbat 80 oder Kombinationen davon ist.

4. Verfahren nach Anspruch 1, wobei der feste Paliperidonester Paliperidonpalmitat ist, das mindestens eine Suspendiermittel Polyethylenglykol 4000 ist und das mindestens eine Benetzungsmittel Polysorbat 20 ist.

5. Verfahren nach einem der Ansprüche 1 bis 4, wobei die Suspension 3 % (Gew./Vol.) ±5 % bis 60 % (Gew./Vol.) ±5 % des festen Paliperidonesters, 2 % (Gew./Vol.) ±5 % bis 30 % (Gew./Vol.) ±5 % des mindestens einen Suspendiermittels und 0,1 % (Gew./Vol.) ±5 % bis 10 % (Gew./Vol.) ±5 % des mindestens einen Benetzungsmittels umfasst.

6. Verfahren nach einem der Ansprüche 1 bis 5, wobei die Vielzahl von Polymerkügelchen aus Polystyrolkügelchen besteht, die mit Divinylbenzol vernetzt sind, und die Vielzahl von Polymerkügelchen in einer Menge von 40% (w/v) ±5% bis 85% (w/v) ±5% vorhanden ist.

7. Verfahren nach einem der Ansprüche 1 bis 6, wobei die Vielzahl der Polymerkügelchen einen durchschnittlichen Durchmesser von 0,6 mm ±5% bis 0,8 mm ±5% aufweist.

8. Verfahren nach einem der Ansprüche 1 bis 7, wobei die Suspension ferner mindestens einen Puffer umfasst, der aus Natriumdihydrogenphosphat, Dinatriumhydrogenphosphat, Natriumacetat, Natriumphthalat oder Kombinationen davon ausgewählt ist, und der mindestens eine Puffer in einer Menge von 0,1 % (w/v) ± 5 % bis 10 % (w/v) ± 5 % vorhanden ist.

9. Verfahren nach einem der Ansprüche 1 bis 8, wobei die Suspension ferner mindestens einen pH-Modifikator umfasst, der aus Salzsäure, Zitronensäure, Natriumhydroxid oder Kombinationen davon ausgewählt ist, und der mindestens eine pH-Modifikator in einer Menge von 0,1 % (w/v) ± 5 % bis 10 % (w/v) ± 5 % vorhanden ist.

10. Verfahren nach einem der Ansprüche 1 bis 9, wobei der mikronisierte Paliperidonester eine Teilchengrößenverteilung aufweist, in der d10 von 2 µm ±5% bis 3 µm ±5%, d50 von 6 µm ±5% bis 8 µm ±5% und d90 von 15 µm ±5% bis 20 µm ±5% beträgt.

11. Verfahren nach einem der Ansprüche 1 bis 10, wobei das Verfahren als kontinuierliches Verfahren durchgeführt wird.

12. Verfahren nach einem der Ansprüche 1 bis 11, wobei das Verfahren aseptisch durchgeführt wird.

13. Verfahren nach einem der Ansprüche 1 bis 12, ferner umfassend die Formulierung des mikronisierten Paliperidonesters in einer injizierbaren Dosierungsform.

## Revendications

1. Procédé en une étape destiné à préparer un ester de palipéridone micronisé, le procédé comprenant un bnumberroyage humide de 5 minutes ±5 % à 60 minutes ±5 % d'une suspension comprenant un ester de palipéridone solide présentant une taille moyenne de particules comprise entre 10 µm ±5 % et 200 µm ±5 %, au moins un agent de suspension, et au moins un agent mouillant en présence d'une pluralité de billes de polymère pour former l'ester de palipéridone micronisé, dans lequel la pluralité de billes de polymère présente un diamètre moyen compris entre 0,5 mm ±5 % et 1,5 mm ± 5 %, l'ester de palipéridone micronisé présente une distribution granulométrique comprise entre 1 µm ±5 % et 30 µm ±5 %, et dans lequel l'au moins un agent de suspension est un polyéthylène glycol, un polypropylène glycol, ou des combinaisons de ceux-ci.

2. Procédé selon la revendication 1, dans lequel l'ester de palipéridone solide est la palipéridone palmitate.

3. Procédé selon la revendication 1 ou la revendication 2, dans lequel l'au moins un agent mouillant est le laurate de polyéthylène glycol, le laurate de glycérol, le stéarate de glycérol, l'oléate de glycérol, le polysorbate 20, le polysorbate 80, ou des combinaisons de ceux-ci.

4. Procédé selon la revendication 1, dans lequel l'ester de palipéridone solide est la palipéridone palmitate, l'au moins un agent de suspension est le polyéthylène glycol 4000, et l'au moins un agent mouillant est le polysorbate 20.

5. Procédé selon l'une quelconque des revendications 1 à 4, dans lequel la suspension comprend 3 % (p/v) ±5 % à 60 % (p/v) ±5 % de l'ester de palipéridone solide, 2 % (p/v) ±5 % à 30 % (p/v) ±5 % de l'au moins un agent de suspension, et 0,1 % (p/v) ±5 % à 10 % (p/v) ±5 % de l'au moins un agent mouillant.

6. Procédé selon l'une quelconque des revendications 1 à 5, dans lequel la pluralité de billes de polymère est constituée de billes de polystyrène réticulées avec du divinylbenzène, et la pluralité de billes de polymère est présente dans une quantité comprise de 40 % (p/v) ±5 % à 85 % (p/v) ±5 %.

7. Procédé selon l'une quelconque des revendications 1 à 6, dans lequel la pluralité de billes de polymère présente un diamètre moyen compris entre 0,6 mm ±5 % et 0,8 mm ±5 %.

8. Procédé selon l'une quelconque des revendications 1 à 7, dans lequel la suspension comprend en outre au moins un tampon choisi parmi le dihydrogénophosphate de sodium, l'hydrogénophosphate disodique, l'acétate de sodium, le phtalate de sodium, ou des combinaisons de ceux-ci, et l'au moins un tampon est présent dans une quantité comprise de 0,1 % (p/v) ±5 % à 10 % (p/v) ±5 %.

9. Procédé selon l'une quelconque des revendications 1 à 8, dans lequel la suspension comprend en outre au moins un modificateur de pH choisi parmi l'acide chlorhydrique, l'acide citrique, l'hydroxyde de sodium, ou des combinaisons de ceux-ci, et l'au moins un modificateur de pH est présent dans une quantité comprise de 0,1 % (p/v) ±5 % à 10 % (p/v) ±5 %.

10. Procédé selon l'une quelconque des revendications 1 à 9, dans lequel l'ester de palipéridone micronisé présente une distribution granulométrique dans laquelle d10 est compris de 2 µm ±5 % à 3 µm ±5 %, d50 est compris de 6 µm ±5 % à 8 µm ±5 %, et d90 est compris de 15 µm ±5 % à 20 µm ±5 %.

11. Procédé selon l'une quelconque des revendications 1 à 10, dans lequel le procédé est mis en œuvre sous la forme d'un procédé en continu.

12. Procédé selon l'une quelconque des revendications 1 à 11, dans lequel le procédé est mené de manière aseptique.

13. Procédé selon l'une quelconque des revendications 1 à 12, comprenant en outre une formulation de l'ester de palipéridone micronisé sous une forme posologique injectable.
